# FASCICULE DE BREVET EUROPEEN

(11) **EP 0 805 706 B1**
(45) Date de publication et mention de la délivrance du brevet: **10.05.2000**
(21) Numéro de dépôt: 96901841.5
(22) Date de dépôt: 24.01.1996
(51) Int. Cl.: A61N 1/30

(54) **DISPOSITIF D'IONOPHORESE POUR L'ADMINISTRATION TRANSCUTANEE D'UN PRINCIPE ACTIF DE TYPE OLIGOSACCHARIDE ANIONIQUE**
IONTOPHORESEVORRICHTUNG ZUR TRANSDERMALEN VERABREICHUNG VON WIRKSTOFFEN VOM TYP ANIONISCHE OLIGOSACCARIDE
IONTOPHORESIS DEVICE FOR THE TRANSCUTANEOUS DELIVERY OF AN ACTIVE PRINCIPLE SUCH AS AN ANIONIC OLIGOSACCHARIDE

(30) Priorité: 24.01.1995 FR 9500757
(43) Date de publication de la demande: 12.11.1997
(73) Titulaire: SANOFI, 75008 Paris (FR); ELF AQUITAINE, 92400 Courbevoie (FR); Akzo Nobel N.V., 6824 BM Arnhem (NL)
(72) Inventeur: MULLER, Daniel, F-64000 Pau (FR); BARBIER, Alain, F-34980 Saint-Clément-de-Rivière (FR); SAUNAL, Henry, F-34000 Montpellier (FR)
(74) Mandataire: Boillot, Marc
(86) Numéro de dépôt international: FR9600114
(87) Numéro de publication internationale: WO9622808

(56) Documents cités:
- EP-A- 0 301 618
- EP-A- 0 498 353
- EP-A- 0 556 112

## Description

L'invention concerne un dispositif d'ionophorèse pour l'administration transcutanée d'un principe actif de médicament de type oligosaccharide anionique et en particulier d'un oligosaccharide anionique de synthèse ayant, entre autres, des activités antithrombotiques et/ou anticoagulantes.

La coagulation sanguine est un phénomène physiologique réputé pour être complexe. Certains stimuli tels que l'activation de contact et les facteurs tissulaires, déclenchent l'activation successive d'une série de facteurs de coagulation présents dans le plasma sanguin.

Quelle que soit la nature du stimulus, les étapes finales sont identiques : le facteur X activé (Xa) active le facteur II (également appelé prothrombine), lequel sous sa forme activée (facteur IIa, également appelé thrombine) provoque la protéolyse partielle du fibrinogène soluble avec libération de la fibrine insoluble, constituant principal du caillot sanguin.

Dans les conditions physiologiques normales, l'activité des facteurs de coagulation est régulée par des protéines telles que l'antithrombine III (AT III) et le cofacteur II de l'héparine (HC II), qui sont également présentes dans le plasma. L'AT III exerce une activité inhibitrice sur un certain nombre de facteurs de coagulation et notamment sur les facteurs Xa et IIa.

L'inhibition du facteur Xa ou du facteur IIa constitue donc un moyen privilégié pour obtenir une activité anticoagulante et antithrombotique puisque ces deux facteurs interviennent dans les deux dernières étapes de la coagulation, lesquelles sont indépendantes du stimulus déclencheur.

Le pentasaccharide de formule (I) 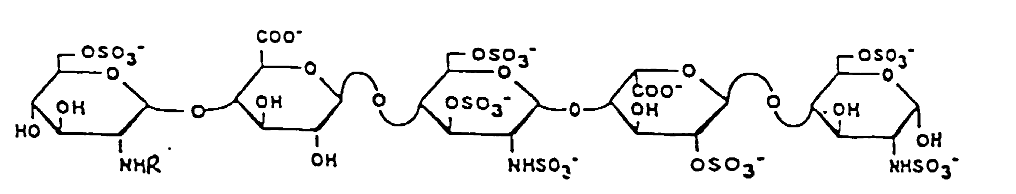 avec R représentant -COCH₃ ou - SO₃⁻ présente une structure appropriée pour la liaison à l'AT III. Ce composé (R = -SO₃⁻) a été obtenu il y a environ une dizaine d'années par synthèse chimique totale (P.Sinaÿ et al, Carbohydrate Research (1984), 132 C5).

Depuis, un certain nombre d'oligosaccharides anioniques de synthèse, obtenus par synthèse chimique totale et ayant des activités antithrombotiques et anticoagulantes, ont été décrits dans la littérature (cf, par exemple, EP-A-0084999, EP-A-0113599, EP-A-0165134, EP-A-0301618, EP-A-0454220 et EP-A-0529715).

Les activités anticoagulantes et antithrombotiques, que peuvent posséder de tels oligosaccharides, font de ces derniers des principes actifs utiles en thérapeutique humaine.

Malheureusement, du fait de leur masse moléculaire assez élevée, de leur forte charge anionique et de leur hydrophilie, ils ne peuvent être administrés par voie orale car ils ne passent pas la barrière gastrointestinale et ils sont essentiellement administrables par voie parentérale, par exemple, voie sous-cutanée ou voie intra-veineuse.

On sait en pareil cas qu'une alternative à la voie parentérale, serait la voie transdermique puisque les composés n'ont pas à traverser le tractus gastrointestinal. Mais on a pu constater que les oligosaccharides du type précité ne pénètrent pas dans la peau avec une vitesse suffisante pour que les concentrations systémiques atteignent des valeurs thérapeutiques efficaces.

On sait que l'ionophorèse peut permettre d'administrer à un sujet, par voie transcutanée, certains principes actifs, généralement consistant en composés de faibles poids moléculaires à caractères ionique.

Pour ce faire, on opère à partir d'une solution aqueuse ou d'un gel aqueux renfermant le principe actif sous une forme au moins partiellement ionisée, en appliquant un signal électrique entre, d'une part, une première électrode, dite électrode active, ayant même polarité que les ions du principe actif à administrer et se trouvant en contact avec un élément réservoir, qui renferme le principe actif et se trouve placé au contact d'une première zone de la peau du sujet, et d'autre part, une deuxième électrode dite contre-électrode ou électrode passive, de polarité opposée à celle associée au principe actif, qui est placée, directement ou par le biais d'un électrolyte indifférent, au contact d'une deuxième zone de la peau du sujet distincte de la première zone. Lors du passage du courant, généré par application du signal électrique entre les électrodes, dans le circuit ainsi réalisé, les ions du principe actif migrent, à l'opposé de l'électrode de même polarité (électrode active), à travers la peau et les tissus du sujet, vers l'électrode de polarité opposée (contre-électrode) et se retrouvent ainsi à passer dans le système circulatoire du sujet.

En étudiant la possibilité d'un passage transcutané de dérivés du type des oligosaccharides à caractère anionique par la technique d'ionophorèse, en faisant appel à des électrodes réutilisables habituellement employées en ionophorèse telles que les électrodes en carbone, platine ou titane, les demanderesses ont observé que les flux transcutanés obtenus pour lesdit dérivés étaient très inférieurs aux flux qu'il conviendrait d'atteindre pour correspondre à une administration thérapeutique.

La citation EP-A-0556112 concerne un dispositif d'ionophorèse pour l'administration transcutanée d'un principe actif de médicament, notamment de type anionique, qui comporte un ensemble électrode négative constitué d'une électrode négative, dite électrode active, en contact avec un élément réservoir contenant un électrolyte renfermant le principe actif sous une forme au moins partiellement ionisée, ledit élément réservoir étant agencé pour assurer, lorsqu'il est placé au contact d'une zone de la peau d'un sujet, un continuum conducteur ionique entre ladite électrode négative et ladite zone, un ensemble électrode positive constitué soit (i) d'une électrode positive seule ou bien, de préférence, (ii) d'une électrode positive en contact avec un élément réceptacle contenant au moins un électrolyte, ledit élément réceptacle étant agencé pour assurer, lorsqu'il est placé en contact d'une portion de la peau du sujet, un continuum conducteur ionique entre l'électrode positive et ladite portion, et un générateur de signaux électriques connectable aux deux électrodes, l'électrode négative en contact avec l'élément réservoir étant formée, au moins en partie, d'un composé métallique ionisable, dont les ions métalliques sont susceptibles d'être réduits électrochimiquement en le métal correspondant et de former avec ledit métal un système réversible électrochimiquement, de manière à constituer, au moins au cours du fonctionnement du dispositif, une électrode négative réversible et le générateur étant agencé pour appliquer entre les électrodes des signaux électriques de tension moyenne telle que la densité du courant moyen généré entre les électrodes soit comprise entre 0,03 et 0,5 mA/cm².

La mise en oeuvre du dispositif d'ionophorèse de la citation EP-A-0556112 avec des principes actifs de type anionique est illustrée essentiellement par l'utilisation d'un produit de masse moléculaire peu élevée, à savoir le valproate de sodium, en concentration allant de 5 % à 15 % en poids, soit 0,3 molaire à 0,9 molaire, ce qui représente plus de 50 mg par cm² d'électrode et par mAh de courant passant par cm² d'électrode.

On sait que, lorsque la masse moléculaire du principe actif augmente, la diffusion et le nombre de transport des ions du principe actif diminuent par rapport à la diffusion et au nombre de transport des ions concurrents, notamment ions Cl⁻ que l'on crée à l'électrode négative (cathode) à base d'AgCl. Si l'on veut obtenir un passage significatif du principe actif par unité de surface d'électrode avec des densités de courant tolérables par la peau, il faut augmenter les concentrations en principe actif et donc la quantité totale du principe actif dans le réservoir de l'électrode avec comme inconvénient de laisser dans ledit réservoir une quantité non consommée du principe actif pouvant aller jusqu'à plus de 99 % de la quantité initiale. En outre, le phénomène d'électroosmose, qui se produit lors de tout processus d'ionophorèse, génère un courant d'eau dans la peau qui circule de l'électrode positive vers l'électrode négative et s'oppose d'autant plus fortement au déplacement des ions de principe actif anionique que la taille desdits ions est plus grande, c'est-à-dire que la masse moléculaire du principe actif est plus élevée.

Il n'était donc pas évident de pouvoir réaliser une administration transcutanée d'un principe actif anionique de masse moléculaire substantiellement plus élevée que celle des produits de type valproate à l'aide d'un dispositif d'ionophorèse tel que décrit dans la citation EP-A-0556112, qui concilierait des flux transcutanés ionophorétiques acceptables de principe actif anionique et un taux d'utilisation significatif du principe actif initialement présent dans le réservoir associé à l'électrode négative.

Les demanderesses ont mis en évidence que l'on pouvait faire appel à un dispositif d'ionophorèse à électrode négative réversible d'un type comparable à celui décrit dans la citation EP-A-0556112, pour réaliser une administration transcutanée de dérivés du type des oligosaccharides anioniques, avec obtention de flux transcutanés ionophorétiques atteignant des concentrations plasmatiques ayant des valeurs compatibles avec un traitement thérapeutique et simultanément utilisation significative du principe actif, si l'on appliquait entre les électrodes des signaux électriques de tension moyenne telle que la densité du courant moyen généré entre lesdites électrodes ait une valeur allant de 0,05 à 0,25 mA/cm² et si le principe actif oligosaccharidique était présent initialement dans l'élément réservoir associé à l'électrode négative en quantité substantiellement inférieure à celle enseignée par la citation EP-A-0556112 pour les principes actifs anioniques de type valproate.

Le dispositif d'ionophorèse selon l'invention comporte un ensemble électrode négative constitué d'une électrode négative, dite électrode active, en contact avec un élément réservoir contenant un électrolyte renfermant le principe actif sous une forme au moins partiellement ionisée, ledit élément réservoir étant agencé pour assurer, lorsqu'il est placé au contact d'une zone de la peau d'un sujet, un continuum conducteur ionique entre ladite électrode négative et ladite zone, un ensemble électrode positive constitué soit (i) d'une électrode positive seule ou bien, de préférence, (ii) d'une électrode positive en contact avec un élément réceptacle contenant au moins un électrolyte, ledit élément réceptacle étant agencé pour assurer, lorsqu'il est placé au contact d'une portion de la peau du sujet, un continuum conducteur ionique entre l'électrode positive et ladite portion, et un générateur de signaux électriques connectable aux deux électrodes, l'électrode négative en contact avec l'élément réservoir étant formée au moins en partie d'un composé métallique ionisable, dont les ions métalliques sont susceptibles d'être réduits électrochimiquement en le métal correspondant et de former avec ledit métal un système réversible électrochimiquement, de manière à constituer, au moins au cours du fonctionnement du dispositif, une électrode négative réversible et le générateur étant agencé pour appliquer, entre les électrodes, des signaux électriques de tension moyenne telle que la densité du courant moyen généré entre lesdites électrodes est comprise entre 0,03 et 0,5 mA/cm², lequel dispositif se caractérise en ce que le principe actif présent dans l'élément réservoir associé à l'électrode négative est choisi parmi les oligosaccharides anioniques représentés par les sels alcalins ou alcalinoterreux d'oligosaccharides qui sont constitués de deux à douze motifs saccharidiques, dont certains motifs ou tous ont leurs groupements OH remplacés, au moins en partie, par des groupements fonctionnels choisis parmi -OSO₃⁻, -COO⁻, -NHSO₃⁻, -NH-acyle, -OPO₃⁻⁻ et -OT, T représentant un radical hydrocarboné, et qui présentent un caractère ionique propre à une administration ionophorétique, en ce que ladite densité de courant possède des valeurs allant de 0,05 mA/cm² à 0,25 mA/cm² et en ce que la quantité de principe actif présente initialement dans l'élément réservoir associé à l'électrode négative représente 0,5 à 12 mg par cm² d'électrode et par mAh de courant passant par cm² d'électrode.

Parmi les composés métalliques susceptibles de constituer au moins en partie l'électrode négative, on peut citer, à titre non limitatif, les composés AgCl et CuCl.

En particulier, on peut constituer l'électrode négative en associant le composé métallique au métal lui correspondant.

La matière de l'électrode négative peut être déposée sur un support, lequel support peut consister en un matériau isolant et notamment en un matériau plastique isolant tel que polypropylène, polyéthylène, PVC, polyester ou bien en un matériau conducteur électronique métallique ou non métallique résistant à la corrosion par l'électrolyte renfermant le principe actif en l'absence de courant comme, par exemple, argent, titane, platine, acier inoxydable, carbone, graphite, polymère conducteur.

L'électrode positive que l'on utilise dans le procédé de l'invention peut être en un métal ou alliage métallique tel que titane, platine, acier inoxydable ou encore en un matériau conducteur électronique non métallique tel que carbone, graphite, polymère conducteur. On peut encore constituer l'électrode positive, au moins en partie, par un métal susceptible d'être consommé par oxydation électrochimique et, par exemple, par un métal tel que Al, Cu, Mg, Zn et Ag. Dans ce cas, on peut en particulier choisir ledit métal consommable par oxydation électrochimique parmi ceux, tels que l'argent, susceptibles de former un système réversible électrochimiquement avec les ions métalliques résultant de l'oxydation électrochimique, de manière à constituer une électrode positive réversible au cours du fonctionnement du dispositif. Le matériau de l'électrode positive consommable par oxydation électrochimique peut être déposé sur un support consistant en un matériau isolant et notamment en un matériau plastique isolant tel que polypropylène, polyéthylène, PVC, polyester ou bien encore en un matériau conducteur électronique métallique ou non métallique tel que, par exemple, titane, platine, acier inoxydable, carbone, graphite, polymère conducteur.

L'électrode négative ou/et l'électrode positive peuvent être agencées pour constituer des électrodes composites formées d'une compositon à base d'un liant polymère, d'une charge conductrice pulvérulente ou fibreuse, notamment noir de carbone ou fibres courtes de graphite, et du matériau actif de l'électrode sous forme divisée, à savoir, dans le cas de l'électrode négative, composé métallique électrochimiquement réductible seul ou associé au métal correspondant, et, dans le cas de l'électrode positive, métal ou alliage métallique choisi pour constituer ladite électrode. Le liant polymère est de préférence un polymère à base d'époxy-1,2 propane et/ou d'époxy-1,2 butane comme décrit dans la demande de brevet français N° 94 09231 déposée le 26.07.1994 par ELF AQUITAINE et SANOFI.

Selon une forme de réalisation du dispositif d'ionophorèse selon l'invention, qui permet de réaliser l'administration transcutanée d'une quantité totale donnée du principe actif du type oligosaccharide anionique à un sujet, l'une ou l'autre des électrodes négative et positive est agencée pour constituer une électrode, dite électrode limitante, formée d'une quantité limitée d'une matière consommable électrochimiquement associée soit à un support conducteur électronique, soit à un support isolant, ladite matière consommable électrochimiquement étant soit le composé métallique électrochimiquement réductible lorsque l'électrode limitante est l'électrode négative ou bien un métal consommable par oxydation électrochimique, notamment un métal tel que Al, Mg, Zn et Ag, lorsque l'électrode limitante est l'électrode positive, et ledit support conducteur électronique étant réalisé en un matériau qui résiste à la corrosion par l'électrolyte associé à l'électrode limitante en l'absence de courant et qui présente, lorsque l'électrode limitante est l'électrode négative, une surtension d'hydrogène en présence dudit électrolyte au moins égale à celle de l'aluminium ou bien qui n'est pas consommable par oxydation électrochimique lorsque l'électrode limitante est l'électrode positive, tandis que ladite quantité limitée de matière consommable électrochimiquement est choisie pour que la quantité d'électricité nécessaire à sa consommation électrochimique corresponde à la quantité d'électricité nécessaire pour administrer la quantité totale donnée de principe actif au sujet, de telle sorte que la circulation du courant entre les électrodes soit pratiquement interrompue lorsque la matière consommable de l'électrode limitante a été consommée, et le principe actif de type oligosaccharide anionique est présent initialement dans l'élément réservoir au contact de l'électrode négative, en quantité supérieure à la quantité totale donnée à administrer au sujet.

Convient en particulier comme support isolant de l'électrode limitante, un support en un matériau plastique isolant tel que polypropylène, PVC, polyéthylène, polyester.

Comme support conducteur électronique de l'électrode négative limitante, on peut choisir avantageusement un support en un matériau choisi parmi aluminium, argent, titane, tantale, vanadium, acier inoxydable, zinc, carbone, graphite et polymère conducteur. Convient, par exemple,comme support de l'électrode positive limitante un support en un matériau choisi parmi platine, titane, acier inoxydable, or, carbone, graphite et polymère conducteur.

Les supports conducteurs métalliques des électrodes négative ou positive peuvent être massifs ou consister en dépôts métalliques de très faible épaisseur sur des films plastiques isolants. Ces dépôts métalliques peuvent être réalisés par toute technique connue telle que, par exemple, métallisation sous vide ou pulvérisation cathodique.

A titre d'exemples non limitatifs d'électrodes utilisables comme électrodes négatives non limitantes ou limitantes dans le dispositif selon l'invention, on peut citer des électrodes à base d'AgCl ou de CuCl sur un support d'argent, de cuivre, d'acier inoxydable, de carbone, de polypropylène, de polyéthylène ou d'un polymère conducteur. Comme exemples d'électrodes positives consommables non limitantes ou limitantes utilisables dans le dispositif selon l'invention, on peut mentionner, à titre non limitatif, les électrodes non limitantes à base d'un métal consommable par oxydation électrochimique choisi parmi Al, Ag, Cu, Mg et Zn et les électrodes limitantes à base d'un tel métal déposé sur un support isolant tel que polypropylène ou polyester ou sur un support choisi parmi titane, acier inoxydable, platine, carbone, graphite et polymère conducteur.

Comme indiqué précédemment, la matière consommable électrochimiquement de l'électrode limitante est présente dans ladite électrode en quantité telle que la quantité d'électricité nécessaire à sa consommation électrochimique corresponde à la quantité d'électricité à utiliser pour administrer la quantité totale donnée du principe actif de type oligosaccharide anionique au sujet. Cette dernière quantité d'électricité, qui dépend du système ionophorétique utilisé, c'est-à-dire des milieux réactionnels en contact avec l'électrode négative réversible et l'électrode positive, du signal électrique appliqué aux électrodes et de la nature desdites électrodes, est déterminée par le biais d'essais préalables pour chaque type de système ionophorétique mis en oeuvre.

Le générateur électrique applique -entre l'électrode négative (électrode active) et l'électrode positive (contre-électrode) un signal électrique qui peut être soit un signal intensiométrique, c'est-à-dire un signal d'intensité moyenne imposée, par exemple constante (signal intensiostatique), soit, de préférence, un signal potentiométrique, c'est-à-dire un signal de tension moyenne imposée, par exemple constante (signal potentiostatique). Le signal électrique de type intensiométrique ou de type potentiométrique peut être continu ou pulsé et permanent ou intermittent, avec ou sans inversion temporaire de polarité. Sa fréquence peut aller de O à 500 kHz et plus particulièrement de 0 à 100 kHz. Lorsque le signal électrique est d'un type pulsé, il peut avoir un rapport cyclique, c'est-à-dire un rapport entre la durée de l'impulsion élémentaire, dont la répétition forme le signal pulsé, et l'intervalle de temps séparant deux apparitions consécutives de cette impulsion, allant de 0,05 à 0,95 et plus particulièrement de 0,1 à 0,8.

Avantageusement, la tension moyenne du signal appliqué par le générateur entre l'électrode négative et l'électrode positive est choisie entre 0,1 et 50 volts et plus spécialement entre 0,3 et 20 volts de telle sorte que la densité du courant moyen généré entre lesdites électrodes ait une valeur comprise entre 0,05 et 0,25 mA/cm² et de préférence entre 0,05 et 0,2 mA/cm².

Le générateur de signaux électriques du dispositif selon l'invention peut être de tout type connu permettant de générer des signaux électriques d'intensité moyenne imposée ou de tension moyenne imposée, qui sont continus ou pulsés et permanents ou intermittents, avec ou sans inversion temporaire de polarité, et qui présentent les caractéristiques définies ci-dessus.

L'électrolyte, qui est présent dans l'élément réservoir en contact avec l'électrode négative, contient avantageusement une solution aqueuse ou un gel aqueux, adhésif ou non, qui renferme le principe actif de type oligosaccharide anionique à administrer sous la forme d'un sel au moins partiellement ionisé d'un métal alcalin tel que, par exemple, sodium ou potassium, ou sel d'un métal alcalino-terreux tel que, par exemple, calcium. De même, l'électrolyte, qui est éventuellement en contact avec l'électrode positive, se présente, au moins en partie, sous la forme d'une solution aqueuse ou d'un gel aqueux adhésif ou non. Ces solutions ou gels aqueux peuvent constituer la totalité de l'électrolyte présent dans l'élément réservoir considéré ou bien peuvent former une partie seulement desdits électrolytes et être alors dispersés dans un milieu non aqueux formant le reste de l'électrolyte et choisi pour ne pas interrompre le continuum conducteur ionique entre l'électrode et la peau et pour accroître la qualité de l'adhésion entre l'électrode et la peau. Ces solutions aqueuses ou gels aqueux peuvent être obtenus comme il est bien connu dans les techniques d'ionophorèse. Des exemples de gels aqueux ou de solutions aqueuses épaisses sont notamment décrits respectivement dans les citations US-A-4766164 et US-A-3163166.

Le milieu aqueux renfermant le principe actif du type oligosaccharide anionique, de même que le milieu aqueux constituant l'électrolyte associé à l'électrode positive, lorsque ledit électrolyte est utilisé, peuvent renfermer, si besoin est, des agents susceptibles de favoriser le passage transcutané du principe actif comme, par exemple, des agents vasodilatateurs et/ou des agents amphiphiles parmi lesquels on peut citer, à titre non limitatif, des composés du type alcool ou du type ester. Ces agents sont utilisés en concentrations permettant une bonne solubilité du principe actif dans le milieu.

Comme indiqué précédemment, la quantité de principe actif de type oligosaccharide anionique présente initialement dans l'élément réservoir associé à l'électrode négative représente 0,5 mg à 12 mg et de préférence 1 mg à 8 mg par cm² de ladite électrode et par mAh de courant passant par cm² de cette électrode.

On peut introduire le principe actif à administrer non seulement dans l'élément réservoir associé à l'électrode négative, mais également dans l'élément réceptacle associé à l'électrode positive et dans ce cas l'électrode négative et l'électrode positive du dispositif d'ionophorèse consistent en une électrode réversible à base du composé métallique ionisable réductible et du métal lui correspondant, par exemple une électrode réversible à base du couple Ag/AgCl. Ceci permet, par inversion de la polarité des signaux électriques appliqués aux électrodes, d'administrer le principe actif alternativement à partir de l'élément réservoir et de l'élément réceptacle.

Dans ce cas, la quantité de principe actif de type oligosaccharidique anionique présente initialement dans l'élément réservoir associé à chacune des électrodes représente 0,5 mg à 6 mg et de préférence 0,5 mg à 4 mg par cm² d'électrode et par mAh de courant passant par cm² d'électrode.

Les oligosaccharides anioniques concernés par l'invention, consistent en sels alcalins ou alcalino-terreux, notamment sels de sodium, potassium ou calcium, de composés qui sont constitués de deux à douze motifs, et plus particulièrement de trois à huit motifs saccharidiques, dont certains motifs ou tous ont leurs groupements OH au moins en partie remplacés par des groupements fonctionnels tels que, par exemple, -OSO₃⁻, -COO⁻, -NHSO₃⁻, -NH acyle, -OPO₃⁻⁻, -OT où T représente un radical hydrocarboné et notamment un radical hydrocarboné aliphatique ou aromatique, -OT étant en particulier un groupement fonctionnel alcoxy, et qui présentent un caractère ionique propre à une administration ionophorétique. Lesdits oligosaccharides sont plus particulièrement des oligosacccharides anioniques obtenus par synthèse chimique totale.

En particulier, lesdits oligosaccharides peuvent être choisis parmi :
- les oligosaccharides de synthèse décrits dans la citation EP-A-0084999, qui sont constitués de 2 à 12 motifs monosaccharides alternés acides uroniques (glucuronique ou iduronique) et glucosamine et renferment, en dehors des groupes OH, des groupements fonctionnels, -OSO₃⁻, -NHSO₃⁻ et -N-acyle, notamment -N-acétyle et, dans certains cas, des groupes alcoxy, notamment méthoxy, en remplacement des groupes anomériques OH. Des exemples de tels oligosaccharides sont les pentasaccharides à propriétés antithrombotiques et/ou anticoagulantes, parmi lesquels on trouve les composés représentés par la formule (I) donnée précédement ;
- les oligosaccharides de synthèse à activité antithrombotique décrits dans la citation EP-A-0165134, qui sont consitués de motifs monosaccharidiques acides uroniques et glucosamine et contiennent des groupements fonctionnels -OSO₃⁻ et -O-PO₃⁻⁻ ;.
- les pentasaccharides à propriétés antithrombotiques et/ou anticoagulantes décrits dans la citation EP-A-0301618, qui sont constitués de motifs acides uroniques et de motifs gluosamine et comportent un groupe -OSO₃⁻ en position 3 de l'unité glucosamine ;
- les oligosaccharides de synthèse à propriétés antithrombotiques et/ou anticoagulantes décrits dans la citation EP-A-0454220, qui sont des dérivés d'acides uroniques et de glucose possédant un enchaînement trisaccharidique spécifique et comportant des groupements fonctionnels O-alkyle ou -O-SO₃⁻ ;
- les dérivés glycosaminoglycanoides sulfatés de synthèse, à propriétés antithrombotiques et à activité inhibitrice de la prolifération des cellules musculaires lisses, décrits dans la citation EP-A-0529715, pour lesquels les groupements fonctionnels -NHSO₃⁻, N-acétate ou OH ont été remplacés par des groupements alcoxy, aryloxy, aralkyloxy ou -O-SO₃⁻ ;
- les héparinoides de synthèse dérivés 3-désoxy à activité antithrombotique décrits dans la demande de brevet français N° 93 04769 déposée le 22.04.1993 par ELF SANOFI et AKZO, qui sont constitués de motifs acides uroniques et de motifs glucosamine et pour lesquels les groupements fonctionnels -NHSO₃⁻, N-acétate du motif glucosamine et éventuellement les groupes OH des motifs acides uroniques et glucosamine ont été remplacés par des groupements alcoxy ou -O-SO₃⁻ ;
- les oligosaccharides de synthèse décrits dans la citation EP-A-0113599, qui sont constitués de motifs monosaccharidiques acides uroniques (glucuronique ou iduronique) et D-galactosamine.

Les oligosaccharides anioniques utilisés selon l'invention sont en particulier des tri-, tétra-, penta- ou hexasaccharides et tout spécialement des pentasaccharides, notamment des pentasaccharides de formule (II) suivante 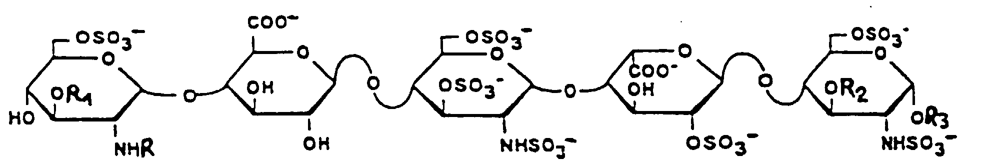 dans laquelle R est un groupement -SO₃⁻ ou acyle, notamment acétyle, R₁ et R₂, identiques ou différents, représentent H ou -SO₃ et R₃ désigne H ou un radical alkyle inférieur, notamment -CH₃.

Le dispositif selon l'invention peut être réalisé à partir de tout dispositif d'ionophorèse connu, que l'on a modifié pour que (i) son électrode négative soit une électrode négative réversible, l'électrode positive étant soit une électrode positive conventionnelle ou bien une électrode positive consommable non réversible ou réversible et l'élément réservoir associé à l'électrode négative renfermant le principe actif du type oligosaccharide anionique sous une forme au moins partiellement ionisée, que (ii) son générateur de signaux électriques soit agencé pour appliquer entre les électrodes des signaux électriques de tension moyenne telle que la densité du courant moyen généré entre les électrodes soit comprise entre 0,05 et 0,25 mA/cm² et de préférence entre 0,05 et 0,2 mA/cm² et que (iii) la quantité de principe actif de type oligosaccharide anionique présente initialement dans l'élément réservoir associé à l'électrode négative ou dans l'élément réservoir ou réceptacle associé à l'électrode négative ou positive ait une valeur telle qu'indiqué précédemment.

Le cas échéant, l'électrode négative ou bien l'électrode positive peut être agencée comme indiqué précédemment pour constituer une électrode limitante.

En particulier, le dispositif selon l'invention peut être un dispositif autonome portable, à fixer par bracelet ou éventuellement à coller sur la peau, comportant des électrodes ayant chacune une aire inférieure à 50 cm² et plus particulièrement comprise entre 1 et 30 cm² et un générateur de signaux électriques miniaturisé. Ainsi, un dispositif portable autonome selon l'invention peut avoir une structure analogue à celle des dispositifs d'ionophorèse portables autonomes décrits, par exemple, dans les citations US-A-4325367, EP-A-0060452 et Fr-A-2509182 sous réserve que l'électrode négative dudit dispositif soit une électrode négative réversible et que le générateur de signaux électriques et l'élément réservoir associé à chaque électrode soient agencés comme indiqué précédemment.

L'électrode négative peut être par exemple une électrode à base d'AgCl ou de CuCl sur un support d'argent, de cuivre, de carbone, de polypropylène, de polyéthylène ou d'un polymère conducteur. L'électrode positive peut être une électrode positive conventionnelle, par exemple électrode en un métal ou alliage métallique tel que titane, platine, acier inoxydable ou encore en un matériau conducteur électronique non métallique tel que carbone ou graphite, ou bien encore une électrode positive consommable non réversible ou réversible, par exemple électrode en un métal tel que Al, Cu, Mg, Zn et Ag éventuellement déposée sur un support isolant tel que polypropylène ou polyester ou sur un support choisi parmi titane, acier inoxydable, platine, carbone, graphite et polymère conducteur. Lesdites électrodes négative et positive, dont l'une et/ou l'autre peuvent être agencées comme indiqué plus haut pour constituer une électrode limitante, ont chacune une aire inférieure à 50 cm² et plus particulièrement comprise entre 1 et 30 cm².

Lorsque l'ensemble électrode négative et l'ensemble électrode positive sont fixés à la peau au moyen d'un adhésif, ceci peut être réalisé en munissant d'une couche d'un adhésif conduisant les ions, la face, destinée à venir au contact de la peau, de l'élément réservoir de chaque ensemble électrode ou une zone entourant ladite face.

Le dispositif d'ionophorèse selon l'invention, lorsqu'il est équipé d'au moins une électrode négative à base du couple Ag/AgCl et, de préférence, comporte une électrode négative et une électrode positive à base dudit couple Ag/AgCl, peut encore inclure un ensemble de contrôle de l'état d'avancement de l'administration transcutanée du principe actif tel que décrit dans la demande de brevet français N° 94 10541 déposée le 2 septembre 1994 par ELF AQUITAINE et SANOFI.

L'invention est illustrée par les exemples suivants donnés à titre non limitatif.

### Exemple 1 :

### Etude du passage transdermique d'un sel de sodium de pentasaccharide par ionophorèse à tension constante.

Le pentasaccharide utilisé correspondait à celui ayant la formule (I) donnée précédemment où R désigne -SO₃⁻.

On opérait dans des cellules d'ionophorèse de structure identique. Chaque cellule d'ionophorèse était constituée de trois compartiments cylindriques adjacents coaxiaux de 2 cm² de section transversale, à savoir, dans cet ordre, un compartiment donneur, un compartiment receveur et un compartiment de contre-électrode, ces trois compartiments étant séparés, chacun du suivant et de façon étanche, par un morceau de peau de rat nu (OFA/hr/hr) servant de membrane pour l'étude de la diffusion trancutanée. Le compartiment donneur, d'un volume de 0,5 ml renfermait une solution aqueuse à 2% en poids du sel de sodium du pentasaccharide précité ayant une activité antifacteur Xa de 0,65 unité "Golden standard" par micro gramme, la quantité de pentasaccharide représentant 10 mg pour 2 cm² de surface active d'électrode. Le compartiment receveur, d'un volume de 10 ml, renfermait du sérum physiologique additionné de 500 ppm de NaN₃ et était agité à l'aide d'un barreau magnétique. Le compartiment de contre-électrode, identique au compartiment donneur, renfermait 0,5 ml d'une solution aqueuse à 2% en poids de chlorure de sodium ainsi que 500 ppm en poids de NaN₃. A son extrémité opposée au compartiment receveur le compartiment donneur était équipé d'une électrode négative. De manière identique au compartiment donneur, le compartiment contre-électrode était équipé d'une électrode positive (contre-électrode).

Les échantillons de peau de rat avaient été débarrassés des tissus sous-cutanés et conservés par congélation à -40°C jusqu'à leur montage dans la cellule d'ionophorèse, faces dermiques tournées vers le compartiment receveur, après un passage de 15 minutes dans du sérum physiologique additionné de 500 ppm de NaN₃.

Pour chacun des essais réalisés, quatre cellules d'ionophorèse identiques étaient mises en route simultanément. La surface active d'échange était de 2 cm² pour chaque peau.

Un générateur de courant pulsé permettait d'établir entre les électrodes des 4 cellules, montées en parallèle, un signal électrique de type potentiostatique de tension crête égale à 2,2 volts avec un rapport cyclique de 50% (soit une tension moyenne de 1,1 volts) et une fréquence de 25 kHz.

Le courant pulsé produit par le générateur était appliqué pendant 6 heures, l'électrode du compartiment donneur de chaque cellule étant reliée au pôle négatif dudit générateur et les contre-électrodes au pôle positif.

Au bout de ladite durée, on prélevait une partie aliquote du milieu contenu dans le compartiment receveur et déterminait par dosage la quantité de pentasaccharide ayant traversé la peau séparant les compartiments donneur et receveur de chaque cellule. Un deuxième prélèvement était réalisé 24 heures après le début de chaque expérience soit 18 heures après l'arrêt du signal électrique.

Cinq essais la à le ont été réalisés comme suit :
- **Essai 1a** : l'électrode négative et l'électrode positive étaient constituées d'un film de titane ayant une épaisseur égale à 10 µm. Aucune tension n'était appliquée aux électrodes dans le but de déterminer la diffusion transcutanée passive.
- **Essai 1b** : L'électrode négative et l'électrode possitive étaient constituées d'une feuille de graphite d'une épaisseur de 60 µm.
- **Essai 1c** : L'électrode négative et l'électrode positive étaient constituées d'un film de titane ayant une épaisseur égale à 10 µm.
- **Essais 1d et 1e** : L'électrode négative était constituée d'un film d'argent de 15 µm d'épaisseur préalablement chloruré sur une face pour renfermer une couche de chlorure d'argent correspondant à 1,8 mAh/cm² tandis que l'électrode positive était constituée d'un film d'argent d'une épaisseur de 15 µm très légèrement chloruré sur une face (quantité de chlorure d'argent correspondant à 0,1 mAh/cm²), la face chlorurée de chaque électrode étant tournée du côté de la membrane en peau de rat.

La chloruration des films d'argent était réalisée électrochimiquement par passage d'un courant continu de 5 mA/cm² alors que chaque film d'argent, dont l'une des faces était protégée par un film plastique adhésif et isolant, était plongé dans un bain d'acide chlorhydrique 0,1 N et constituait le pôle positif par rapport à une électrode en graphite plongée dans le même bain de HCl, la quantité de courant étant contrôlée par un coulomètre monté en série dans le circuit, pour former la quantité désirée de chlorure d'argent sur le film d'argent.

Dans les essais 1a à 1d, les compartiments donneur et receveur de chaque cellule renfermaient une composition tampon 0,06 molaire à base de monohydrogénophosphate et de dihydrogénophosphate de sodium en quantités équimoléculaires, de manière à maintenir le pH dans lesdits compartiments à une valeur d'environ 7. Dans l'essai le, aucun tampon n'était utilisé. Dans les essais ld et le, la densité du courant moyen généré entre les électrodes était égale à environ 0,20 mA/cm².

Pour chacun des essais, on a déterminé sur des parties aliquotes prélevées dans les compartiments receveurs des quatre cellules, l'activité moyenne antifacteur Xa par ml de milieu représentative de la quantité de pentasaccharide ayant diffusé dans ces compartiments à l'issue des 6 heures d'application du courant et 24 heures après le début de chaque expérience, soit 18 heures après l'arrêt dudit courant.

Le dosage du pentasaccharide dans le compartiment récepteur était fondé sur la recherche de son activité antifacteur Xa. Le dosage était réalisé, soit directement sur le milieu prélevé dans le compartiment récepteur, soit après dilution, à l'aide d'une trousse de dosage ROTACHROM HEPARIN 8®, de tampon complémentaire pour appareil de dosage et d'antithrombine III bovine, ces divers éléments étant fournis par la société STAGO, en faisant appel à un appareil de dosage HITACHI 717. L'établissement de la courbe d'étalonnage était effectué à l'aide d'une solution étalon de pentasaccharide dite étalon "Golden Standard" à laquelle on a attribué une activité antifacteur Xa égale à 13 unités par ml.

Les résultats obtenus sont rassemblés dans le tableau I.

**TABLEAU I**

| **ESSAI** | **1a** | **1b** | **1c** | **1d** | **1e** |
|---|---|---|---|---|---|
| Tension moyenne (volts) | 0 | 1,1 | 1,1 | 1,1 | 1,1 |
| Nature de l'électrode négative | Titane | Graphite | Titane | Ag/AgCI | Ag/AgCI |
| Activité antifacteur Xa/ml à 6 heures | <0,1 | 0,70 | 0,50 | 9,1 | 18,1 |
| % variation par rapport à la moyenne | | 38 | 54 | 41 | 46 |
| Taux d'utilisation du principe actif (%) | ~ nul | 0,1 | 0,08 | 1,4 | 2,8 |
| Activité antifacteur Xa/ml à 24 heures | 0,25 | 1,13 | 0,95 | 9,7 | 21,4 |
| % variation par rapport à la moyenne | 60 | 45 | 51 | 43 | 39 |
| Taux d'utilisation du principe actif (%) | ~ nul | 0,2 | 0,15 | 1,5 | 3,3 |

Le niveau de l'activité antifacteur Xa étant le reflet de la concentration du pentasaccharide dans le compartiment receveur, l'examen du tableau I montre clairement que les quantités de pentasaccharide ayant diffusé dans le compartiment receveur par ionophorèse sont très faibles et proches du transport passif lorsque l'on utilise des électrodes non réversibles comme le graphite ou le titane, alors que lesdites quantités augmentent très fortement lorsque l'on fait appel à des électrodes négatives Ag/AgCl (électrodes réversibles) même en présence d'un tampon. Ces électrodes réversibles, à la différence des électrodes non consommables comme le titane ou le graphite, permettent d'éviter l'hydrolyse de l'eau et donc des changements importants de pH en cours de traitement tant au niveau des électrodes négatives que positives. La présence de substances à effet tampon dans les compartiments donneurs n'est donc plus nécessaire, ce qui constitue un avantage supplémentaire des électrodes réversibles. Ainsi qu'on peut le constater à la lecture de l'essai le, les flux transdermiques sous ionophorèse obtenus en l'absence du tampon sont encore accrus par rapport à tous les autres essais. 137 µg/cm2 par mAh).

### Exemple 2 :

### Etude du passage transdermique d'un sel de sodium de pentasaccharide par ionophorèse à intensité imposée

On opérait comme décrit dans l'exemple 1 avec toutefois les modifications suivantes :
- mise en oeuvre de l'ionophorèse en imposant une intensité constante à travers chaque cellule
- utilisation d'un courant continu au lieu de courant pulsé
- absence de tampon dans les compartiments donneurs et de contre-électrode de l'essai 2d car ceux-ci sont équipés d'électrodes de type Ag/AgCl qui évitent l'hydrolyse de l'eau.

La mise en oeuvre était effectuée en imposant aux électrodes de chacune des 4 cellules d'ionophorèse, pendant une durée de 6 heures, un courant continu constant de 0,4 mA, soit 0,2 mA/cm², à l'aide d'un générateur permettant d'imposer une intensité constante quelles que soient les tensions générées aux bornes de chaque cellule.

Le courant continu d'intensité constante égale à 0,4 mA produit par le générateur était appliqué pendant 6 heures, le compartiment donneur de chaque cellule étant relié au pôle négatif dudit générateur et les contre-électrodes au pôle positif.

Pour chacun des essais 2a à 2d on a déterminé sur des parties aliquotes, prélevées dans les compartiments receveurs, l'activité moyenne antifacteur Xa par ml du milieu receveur, représentative de la quantité de pentasaccharide y ayant diffusé à l'issue des 6 heures d'application du courant et 24 heures après le début de chaque expérience, soit 18 heures après l'arrêt dudit courant.

Les résultats obtenus sont rassemblés dans le tableau II

**TABLEAU Il**

| **ESSAI** | **2a** | **2b** | **2c** | **2d** |
|---|---|---|---|---|
| Densité moyenne de courant (mA/cm²) | 0 | 0,2 | 0,2 | 0,2 |
| Nature de l'électrode négative | Titane | Graphite | Titane | Ag/AgCI |
| Activité antifacteur Xa/ml à 6 heures | 0,12 | 6 | 3,2 | 18,1 |
| % variation par rapport à la moyenne | | 35 | 42 | 22 |
| Taux d'utilisation du principe actif (%) | ~ nul | 0,09 | 0,49 | 2,8 |
| Activité antifacteur Xa/ml à 24 heures | 0,6 | 5,8 | 4,9 | 24 |
| % variation par rapport à la moyenne | 60 | 45 | 51 | 18 |
| Taux d'utilisation du principe actif (%) | 0,09 | 0,9 | 0,75 | 3,7 |

Les quantités totales diffusées, exprimées en µg par cm² pour chacun des essais, sont respectivement égales à 4,64 (essai 2a), 45 (essai 2b), 38 (essai 2c) et 185 (essai 2d), soit 154 µg/cm² par mAh pour les électrodes Ag/AgCl.

Bien que, par rapport aux essais précédents conduits à tension constante, la diffusion sous ionophorèse soit devenue plus significative par rapport à la diffusion passive pour les électrodes de graphite et de titane, les performances des électrodes d'argent chloruré restent très nettement supérieures.

### EXEMPLE 3

### Administration chez le microporc du sel de sodium d'un pentasaccharide par ionophorèse à densité de courant imposée

Le microporc de souche YUCATAN est considéré par les spécialistes comme un excellent modèle animal pour l'étude de l'administration chez l'homme des médicaments par ionophorèse. En effet la structure de la peau de cet animal est très voisine de celle de la peau humaine.

Les essais suivants ont été conduits chez le microporc précité dans le but de comparer l'administration du sel de sodium du pentasaccharide utilisé dans les exemples précédents par ionophorèse avec une administration par injection sous-cutanée ou intraveineuse.

Pour ces essais, on constituait des paires d'ensembles électrodes adhésifs, chaque paire comportant un ensemble électrode donneur formé d'une électrode négative réversible (électrode active) en contact avec un premier élément réservoir renfermant le pentasaccharide à administrer et un ensemble électrode passif formé d'une électrode positive (contre-électrode) en contact avec un deuxième élément réservoir (élément réceptacle) renfermant un électrolyte indifférent.

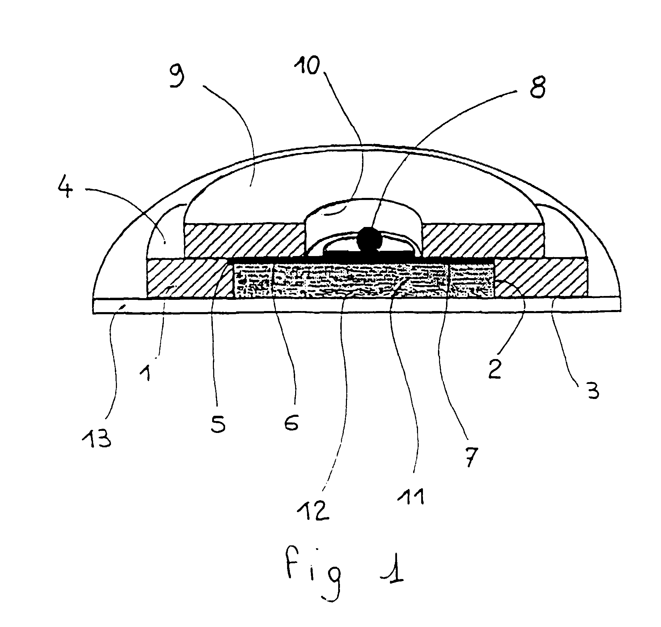

Chaque ensemble électrode avait une structure analogue à celle qui est schématisée, à titre d'exemple non limitatif, sur la figure.

En se référant à la figure, chaque ensemble électrode comportait un disque 1 en mousse de polyéthylène présentant un évidement cylindrique axial 2, ledit disque comportant une face 3 adhésive et une face 4 non-adhésive, chacune desdites faces ayant la forme d'une zone annulaire de deux centimètres de largeur. L'extrémité 5 de l'évidement du disque, côté face non-adhésive, était obturé par une électrode 6 ayant la forme d'un disque d'argent chloruré sur l'une de ses faces, ledit disque ayant une section de 20 cm². La face chlorurée 7 du disque électrode était tournée vers l'intérieur de l'évidement. La face non-chlorurée dudit disque portait une prise de contact 8 du type bouton-pression, soudée à ladite face au moyen d'un adhésif conducteur électronique, et elle s'appuyait contre un disque support 9 en mousse de polyéthylène coaxial au disque 1 et présentant un évidement axial 10 pour permettre l'acès à la prise de contact 8. Ledit disque support, de diamètre compris entre celui de l'électrode 6 et du disque 1, était collé sur la face non-adhésive de ce dernier disque. L'évidement 2 du disque 1 était rempli d'un hydrogel conducteur 11 formant élément réservoir. La face adhésive 3 du disque 1 était enduite d'un adhésif sensible à la pression agréé pour être appliqué sur la peau, ladite face 3 et la face 12 adjacente de l'élément réservoir 11 étant initialement recouvertes par un film protecteur pelable anti-adhérent en polyester, que l'on retirait avant application sur la peau.

Dans l'ensemble électrode donneur, l'électrode négative (électrode active) en argent chloruré renfermait une quantité de chlorure d'argent équivalant à 1,8 mAh/cm2, permettant ainsi à l'électrode de supporter la quantité de courant traversant les électrodes pendant la durée du traitement ionophorétique, à savoir 1,2 mAh/cm². L'élément réservoir associé à l'électrode négative était rempli sur une épaisseur de 2 mm, soit une quantité de 4 g pour les 20 cm² d'électrode, d'un hydrogel à base de xanthane et d'extrait de caroube à 3 % d'extrait sec et contenant 2 % en poids de sel de sodium du pentasaccharide de formule (I) donnée précédemment. L'ensemble électrode donneur contenait donc 80 mg de pentasaccharide à raison de 4 mg/cm² pour chacun des animaux traités.

Dans l'ensemble électrode passif, l'électrode positive (contre-électrode) en argent chloruré renfermait une quantité de chlorure d'argent équivalant à 0,1 mAh/cm2 et l'élément réservoir associé à cette électrode était constitué du même hydrogel que celui présent dans l'ensemble électrode donneur exempt toutefois de pentasaccharide, mais renfermant, par contre, 4 % en poids de NaCl.

Un générateur de signaux électriques, connectable aux électrodes de chaque paire d'ensembles électrodes permettait de délivrer entre lesdites électrodes un signal électrique pulsé d'intensité régulée ayant une fréquence de 25 kHz et un rapport cyclique égal à 50 %.

Cinq jours avant chaque essai, les animaux étaient cathétérisés au niveau des deux jugulaires, ainsi qu'il est bien connu pour toute expérimentation d'administration de médicaments, afin de permettre des prélèvements réguliers de sang destinés à doser les activités antifacteur Xa et, par là, à évaluer les quantités de principe actif ayant traversé la peau pour pénétrer le système circulatoire et donc à vérifier l'efficacité du traitement ionophorétique.

Les animaux, à jeun depuis la veille des expérimentations, étaient placés dans des hamacs spécialisés. On collait par simple pression sur le dos de chaque animal préalablement nettoyé à l'aide d'un tissu humide, de part et d'autre de la colonne vertébrale, une paire d'ensembles électrodes préalablement débarrassés de leur film protecteur pelable et on connectait, par l'intermédiaire de câbles munis de pinces adaptées aux prises de contact installées à cet effet, l'électrode négative de l'ensemble électrode donneur au pôle négatif du générateur et la contre-électrode de l'ensemble électrode passif au pôle positif dudit générateur.

Entre les électrodes positive et négative de chaque paire d'ensembles électrodes collés sur l'animal, on établissait, grâce au générateur, un courant de 4,8 mA (soit une densité de courant de 0,20 mA/cm²) pendant une durée de 6 heures et on pratiquait divers prélèvements de sang sur diatube H STAGO® au cours du temps et ce jusqu'à 30 heures après le début de chaque expérience soit 25 heures après la fin du traitement ionophorétique. L'essai ionophorétique a été reproduit sur cinq microporcs mâles de poids égal à 11,4 kg en moyenne.

Outre les essais d'administration du principe actif par ionophorèse mis en oeuvre comme décrit ci-dessus (essai 3b), on effectuait également des essais comparatifs 3a, 3c et 3d comme suit, chaque essai étant reproduit sur deux à quatre microporcs, comme dans le cas de l'administration ionophorétique :
. essai 3a : mise en place des ensembles électrodes comme dans l'essai d'ionophorèse 3b, mais sans application de courant,
. essai 3c : injection intraveineuse en "bolus" de 0,240 mg/kg de pentasaccharide en solution injectable,
. essai 3d : injection sous-cutanée de 0,200 mg/kg de pentasaccharide en solution injectable.

Dans ces essais 3a, 3c et 3d, on pratiquait un certain nombre de prélèvements sanguins au cours du temps et jusqu'à 30 heures après le début de chaque expérience comme dans le cas du traitement ionophorétique de l'essai 3b.

Les teneurs plasmatiques des différents prélèvements sanguins, exprimées en unités "Golden standard" d'antifacteur Xa, sont indiquées dans le tableau III aux fins de comparaison des taux plasmatiques moyens obtenus sur des animaux de même souche et de même poids traités avec le même principe actif dans les conditions des essais selon l'invention (essai 3b) et comparatifs (essais 3a, 3c et 3d).

**TABLEAU III**

| **ESSAI** | **3a** | **3b** | **3c** | **3d** |
|---|---|---|---|---|
| Nature du traitement | passif | ionophorèse | injection I.V. | injection sous-cutanée |
| Nombre de microporcs traités | 2 | 5 | 4 | 4 |
| Densité de courant (mA/cm²) | 0 | 0,20 | | |
| Taux plasmatique à 0 minute | 0 | 0 | 0 | 0 |
| Taux plasmatique à 5 minutes | 0 | non dosé | 1,04 ± 0,12 | non dosé |
| Taux plasmatique à 15 minutes | 0 | 0,06 ± 0,04 | 0,91 ± 0,13 | 0,11 ± 0,04 |
| Taux plasmatique à 30 minutes | 0 | 0,15 ± 0,12 | 0,65 ± 0,11 | non dosé |
| Taux plasmatique à 1 heure | 0 | 0,31 ± 0,15 | 0,53 ± 0,10 | 0,17 ± 0,01 |
| Taux plasmatique à 2 heures | 0 | 0,45 ± 0,2 | 0,30 ± 0,09 | 0,22 ± 0,04 |
| Taux plasmatique à 4 heures | 0 | 0,64 ± 0,18 | 0,29 ± 0,08 | 0,26 ± 0,06 |
| Taux plasmatique à 6 heures | 0 | 0,74 ± 0,16 | 0,22 ± 0,09 | 0,24 ± 0,01 |
| Taux plasmatique à 8 heures | 0 | 0,52 ± 0,13 | 0,17 ± 0,06 | 0,15 ± 0,07 |
| Taux plasmatique à 12 heures | 0 | 0,4 ± 0,08 | 0,07 ± 0,05 | 0.08 ± 0,03 |
| Taux plasmatique à 24 heures | 0 | 0,11 ± 0,03 | 0 | 0 |
| Taux plasmatique à 30 heures | 0 | 0 | 0 | 0 |

La comparaison des résultats du tableau III fait apparaître que le traitement par voie ionophorétique pratiqué selon l'invention conduit à des taux plasmatiques, exprimés en antifacteur Xa, supérieurs à ceux que l'on peut obtenir avec des injections intraveineuses ou sous-cutanées. Les taux plasmatiques s'élèvent assez rapidement et régulièrement au cours du traitement et décroissent lentement apès arrêt du courant.

L'administration de ce type de principe actif par voie ionophorétique,surtout si celle-ci est étalée sur une durée quotidienne un peu plus longue et avec des densités de courant encore plus faibles, permet d'obtenir une concentration plasmatique de principe actif conduisant à une bonne couverture antithrombotique, exprimée en termes d'antifacteur Xa, sur une durée relativement importante en évitant un passage par des taux plasmatiques de pointe élevés.

La comparaison des aires sous les courbes représentant l'évolution des taux plasmatiques au cours du temps, lors des différents traitements, permet au pharmaco-cinéticien de calculer, en fonction du poids des animaux, les quantités de pentasaccharide qui ont été réellement administrées par ionophorèse. Cette quantité a été estimée dans le cas de l'essai 3b à 7 ± 1,3 mg pour les 20 cm², soit 350 µg/cm² pour 1,2 mAh/cm², soit encore 292 µg/cm² pour 1 mAh/cm².

La biodisponibilité, c'est-à-dire le taux de principe actif réellement administré rapporté aux quantités de principe actif présentes dans les électrodes, est donc d'environ 9 %, ce qui est supérieur aux taux d'utilisation qui ont été observés pour les essais in vitro.

### Exemple 4

### Administration chez le microporc du sel de sodium d'un pentasaccharide par ionophorèse à densité de courant imposée avec inversion périodique du sens du courant.

Les essais suivants d'administration ionophorétique ont été conduits sur 4 microporcs en utilisant le même générateur de courant et le même type d'électrodes que dans l'exemple 3 et en appliquant les conditions suivantes :

Les électrodes de même structure que dans l'exemple 3 avaient une surface active de 20 cm². Les électrodes elles-mêmes étaient constituées de films d'argent, de 15 µm d'épaisseur, chlorurés par oxydation électrolytique à raison de 0,5 mAh de chlorure d'argent par cm². Les réservoirs, également de 20 cm², étaient constitués d'une feuille de papier buvard en fibres de cellulose additionnées de fibrilles de polypropylène, imbibée à raison de 50 mg/cm² d'une solution aqueuse contenant 2 % en poids de pentasaccharide et 0,2 % en poids de NaCl. Chaque électrode, anode et cathode, contenait en tout 1 g de solution, soit 20 mg de pentasaccharide (1 mg/cm²) pour 20 cm² de surface active. On a donc engagé 40 mg de pentasaccharide en tout par animal.

Le générateur de courant délivrait une intensité constante de 2,5 mA, soit 125 µA/cm² de courant continu avec des inversions régulières du sens du courant toutes les 30 minutes pendant une durée totale de 8 heures correspondant au passage de 1 mA/cm² de courant continu.

Chacune des électrodes, de structure et de composition identique, se trouvait donc fonctionner alternativement comme cathode puis comme anode, la présence de chlorure de sodium dans chacun des réservoirs garantissant leur fonctionnement permanent comme électrode réversible, évitant ainsi toute réaction parasite d'hydrolyse (avec changement du pH) ou d'oxydoréduction du principe actif.

Dans l'essai 4a mettant en jeu 3 animaux de poids moyen de 11,8 kg, les électrodes étaient mises en place mais non connectées au générateur de courant alors que pour l'ensemble des essais 4b, qui concernaient 4 animaux de poids moyen de 12,3 kg, le courant de 2,5 mA était appliqué pendant 8 heures avec une inversion automatisée du sens du courant toutes les demi-heures.

On pratiquait, dans les essais 4a et 4b, un certain nombre de prélèvements sanguins au cours du temps jusqu'à 30 heures après le début de chaque expérience, comme dans l'exemple 3.

Les teneurs plasmatiques des différents prélèvements sont indiquées dans le tableau IV aux fins de comparaison des essais 4a et 4b entre eux et avec les résultats des essais réalisés dans l'exemple 3, en particulier les essais 3c et 3d correspondant aux injections -intraveineuses et sous-cutanées.

La comparaison de l'aire sous la courbe représentant l'évolution des taux plasmatiques au cours du temps pour l'essai 4b avec celle des essais 3c et 3d permet au pharmaco-cinéticien d'évaluer avec une bonne précision la quantité moyenne de pentasaccharide administrée au cours du traitement. Cette quantité est nulle pour l'essai passif 4a et de 8, 45 ± 1,5 mg de pentasaccharide pour l'essai 4b.

**TABLEAU IV**

| **ESSAI** | **4a** | **4b** |
|---|---|---|
| Nature du traitement | passif | ionophorèse |
| Nombre d'animaux traités | 2 | 4 |
| Densité de courant (mA/cm²) | 0 | 0,125 |
| Taux plasmatique initial | 0 | 0 |
| Taux plasmatique à 0,5 heure | 0 | 0,11 ± 0,05 |
| Taux plasmatique à 1 heure | 0 | 0,16 ± 0,05 |
| Taux plasmatique à 2 heures | 0 | 0,28 ± 0,1 |
| Taux plasmatique à 4 heures | 0 | 0,64 ± 0,2 |
| Taux plasmatique à 6 heures | 0 | 0,57 ± 0,18 |
| Taux plasmatique à 8 heures | 0 | 0,59 ± 0,15 |
| Taux plasmatique à 10 heures | 0 | 0,61 ± 0,16 |
| Taux plasmatique à 12 heures | 0 | 0,58 ± 0,05 |
| Taux plasmatique à 16 heures | 0 | 0,2 ± 0,05 |
| Taux plasmatique à 26 heures | 0 | 0,14 ± 0,02 |
| Taux plasmatique à 30 heures | 0 | 0,05 ± 0,02 |

Par rapport à l'essai précèdent, pour lequel on avait engagé 80 mg de pentasaccharide, on observe une meilleure biodisponibilité, soit environ 21 % pour l'essai 4b contre environ 9 % pour l'essai 3b. On observe également un meilleur rendement électrique, puisque la quantité de pentasaccharide administrée par mAh était de 292 µg/cm² pour l'essai 3b alors qu'elle est de 372 µg/cm² pour l'essai 4 b.

Cette approche par inversion périodique et équilibrée du courant, qui permet de consommer dans chaque réservoir une partie des ions chlorures générés par la réduction du chlorure d'argent lors de la phase précédente, diminue la compétition des ions chlorures par rapport aux ions thérapeutiques et autorise la diminution des quantités de principe actif par unité de surface. Elle a montré, en outre, l'intérêt d'employer des électrodes réversibles qui empêchent les réactions d'oxydoréduction qui pourraient affecter le principe actif qui se trouve alternativement dans un compartiment anodique puis cathodique.

Les appareils d'ionophorèse pouvant désormais être miniaturisés et d'un port tout à fait compatible avec une vie quotidienne normale, l'ionophorèse pratiquée selon l'invention constitue une voie galénique particulièrement intéressante pour l'administration de pentasaccharides du type précité et plus largement d'oligosaccharides anioniques analogues ou voisins.

## Revendications

1. Dispositif d'ionophorèse pour l'administration transcutanée d'un principe actif de médicament de type anionique, qui comporte un ensemble électrode négative constitué d'une électrode négative, dite électrode active, en contact avec un élément réservoir contenant un électrolyte renfermant le principe actif sous une forme au moins partiellement ionisée, ledit élément réservoir étant agencé pour assurer, lorsqu'il est placé au contact d'une zone de la peau d'un sujet, un continuum conducteur ionique entre ladite électrode négative et ladite zone, un ensemble électrode positive constitué soit (i) d'une électrode positive seule ou bien, de préférence, (ii) d'une électrode positive en contact avec un élément réceptacle contenant au moins un électrolyte, ledit élément réceptacle étant agencé pour assurer, lorsqu'il est placé au contact d'une portion de la peau du sujet, un continuum conducteur ionique entre l'électrode positive et ladite portion, et un générateur de signaux électriques connectable aux deux électrodes, l'électrode négative en contact avec l'élément réservoir étant formée au moins en partie d'un composé métallique ionisable, dont les ions métalliques sont susceptibles d'être réduits électrochimiquement en le métal correspondant et de former avec ledit métal un système réversible électrochimiquement, de manière à constituer, au moins au cours du fonctionnement du dispositif, une électrode négative réversible et le générateur étant agencé pour appliquer, entre les électrodes, des signaux électriques de tension moyenne telle que la densité du courant moyen généré entre lesdites électrodes est comprise entre 0,03 et 0,5 mA/cm², lequel dispositif se caractérise en ce que le principe actif présent dans l'élément réservoir associé à l'électrode négative est choisi parmi les oligosaccharides anioniques représentés par les sels alcalins ou alcalinoterreux d'oligosaccharides qui sont constitués de deux à douze motifs saccharidiques, dont certains motifs ou tous ont leurs groupements OH remplacés, au moins en partie, par des groupements fonctionnels choisis parmi -OSO₃⁻, -COO⁻, -NHSO₃⁻, -NH-acyle, -OPO₃⁻⁻ et -OT, T représentant un radical hydrocarboné, et qui présentent un caractère ionique propre à une administration ionophorétique, en ce que ladite densité de courant possède des valeurs allant de 0,05 mA/cm² à 0,25 mA/cm² et en ce que la quantité de principe actif présente initialement dans l'élément réservoir associé à l'électrode négative représente 0,5 à 12 mg par cm² d'électrode et par mAh de courant passant par cm² d'électrode.

2. Dispositif selon la revendication 1, caractérisé en ce que lesdits oligosaccharides anioniques sont obtenus par synthèse chimique totale.

3. Dispositif selon la revendication 1 ou 2, caractérisé en ce que le composé métallique susceptible de constituer au moins en partie l'électrode négative est choisi parmi les composés AgCl et CuCl.

4. Dispositif selon l'une des revendications 1 à 3, caractérisé en ce que la matière de l'électrode négative est déposée sur un support, lequel support consiste en un matériau isolant et notamment en un matériau plastique isolant tel que polypropylène, polyéthylène, PVC, polyester ou bien en un matériau conducteur électronique métallique ou non métallique résistant à la corrosion par l'électrolyte renfermant le principe actif en l'absence de courant, comme par exemple, argent, titane, platine, acier inoxydable, carbone, graphite, polymère conducteur.

5. Dispositif selon la revendication 4, caractérisé en ce que l'électrode négative est à base du couple Ag/AgCl.

6. Dispositif selon l'une des revendications 1 à 5, caractérisé en ce que l'électrode positive est en un métal ou alliage métallique tel que titane, platine, acier inoxydable ou encore en un matériau conducteur électronique non métallique tel que carbone ou graphite, ou bien consiste au moins en partie, en un métal susceptible d'être consommé par oxydation électrochimique, par exemple métal tel que Al, Cu, Mg, Zn et Ag, ledit métal consommable par oxydation électrochimique étant en particulier choisi parmi ceux susceptibles de former un système réversible électrochimiquement avec les ions métalliques résultant de l'oxydation électrochimique, de manière à constituer une électrode positive réversible.

7. Dispositif selon la revendication 6, caractérisé en ce que l'électrode positive est à base du couple Ag/AgCl.

8. Dispositif selon l'une des revendications 1 à 7, permettant d'administrer une quantité totale donnée du principe actif de type oligosaccharide anionique au sujet, caractérisé en ce que l'une ou l'autre des électrodes négative et positive est agencée pour constituer une électrode, dite électrode limitante, formée d'une quantité limitée d'une matière consommable électrochimiquement associée soit à un support conducteur électronique, soit à un support isolant, ladite matière consommable électrochimiquement étant soit le composé métallique électrochimiquement réductible lorsque l'électrode limitante est l'électrode négative ou bien un métal consommable par oxydation électrochimique, notamment un métal tel que Al, Mg, Zn, et Ag, lorsque l'électrode limitante est l'électrode positive, et ledit support conducteur électronique étant réalisé en un matériau qui résiste à la corrosion par l'électrolyte associé à l'électrode limitante en l'absence de courant et qui présente, lorsque l'électrode limitante est l'électrode négative, une surtension d'hydrogène en présence dudit électrolyte au moins égale à celle de l'aluminium ou bien qui n'est pas consommable par oxydation électrochimique lorsque l'électrode limitante est l'électrode positive, tandis que ladite quantité limitée de matière consommable électrochimiquement est choisie pour que la quantité d'électricité nécessaire à sa consommation électrochimique corresponde à la quantité d'électricité nécessaire pour administrer au sujet la quantité totale donnée de principe actif, de telle sorte que la circulation du courant entre les électrodes soit pratiquement interrompue lorsque la matière consommable de l'électrode limitante a été consommée, et en ce que le principe actif de type oligosaccharide anionique est présent, au début de l'opération, dans l'élément réservoir au contact de l'électrode négative en quantité supérieure à la quantité totale donnée à administrer au sujet.

9. Dispositif selon l'une des revendications 1 à 8, caractérisé en ce que le générateur de signaux électriques applique entre l'électrode négative et l'électrode positive un signal intensiométrique, c'est-à-dire un signal d'intensité moyenne imposée, ou un signal potentiométrique, c'est-à-dire un signal de tension moyenne imposée, ledit signal électrique étant continu ou pulsé et permanent ou intermittent, avec ou sans inversion temporaire de polarité, et possédant une fréquence allant de 0 à 500 kHz et plus particulièrement de 0 à 100 kHz.

10. Dispositif selon la revendication 9, caractérisé en ce que le signal électrique est un signal pulsé présentant un rapport cyclique, c'est-à-dire un rapport entre la durée de l'impulsion élémentaire, dont la répétition forme le signal pulsé, et l'intervalle de temps séparant deux apparitions consécutives de cette impulsion allant de 0,05 à 0,95 et plus particulièrement de 0,1 à 0,8.

11. Dispositif selon la revendication 9 ou 10, caractérisé en ce que le signal appliqué entre l'électrode négative et l'électode positive présente une tension moyenne choisie entre 0,1 et 50 volts et plus spécialement entre 0,5 et 20 volts de telle sorte que la densité du courant moyen généré entre lesdites électrodes ait une valeur comprise entre 0,05 et 0,25 mA/cm² et plus particulièrement entre 0,05 et 0,2 mA/cm².

12. Dispositif selon l'une des revendications 1 à 11, caractérisé en ce que le milieu aqueux renfermant le principe actif de type oligosaccharide anionique ou/et le milieu aqueux constituant l'autre électrolyte renferment des agents susceptibles de favoriser le passage transcutané du principe actif, comme, par exemple, des agents vasodilatateurs ou/et des agents amphiphiles tels que, notamment, des composés du type alcool ou du type ester.

13. Dispositif selon l'une des revendications 1 à 12, caractérisé en ce que le principe actif de type oligosaccharide anionique présent dans l'élément réservoir associé à l'électrode négative est choisi parmi les sels alcalins ou alcalino-terreux, notamment sels de sodium, potassium ou calcium, des oligosaccharides qui sont constitués de trois à huit motifs saccharidiques, dont certains motifs ou tous ont leurs groupements OH au moins en partie remplacés par des groupements fonctionnels tels que, par exemple, -OSO₃⁻, -COO⁻, -NHSO₃⁻, -NH-acyle, -OPO₃⁻⁻, -OT où T représente un radical hydrocarboné et notamment un radical hydrocarboné aliphatique ou aromatique, -OT étant en particulier un groupement alcoxy, et qui présentent un caractère ionique propre à une administration ionophorétique, lesdits oligosaccharides étant plus spécialement des oligosaccharides anioniques obtenus par synthèse chimique totale.

14. Dispositif selon la revendication 13, caractérisé en ce que les oligosaccharides anioniques sont des tri-, tétra-, penta- ou hexasaccharides et tout particulièrement des pentasaccharides.

15. Dispositif selon l'une des revendications 1 à 14, caractérisé en ce que les oligosaccharides anioniques sont constitués de motifs alternés acides uroniques et glucosamine ou de motifs alternés acides uroniques et glucose ou encore de motifs alternés acides uroniques et galactosamine.

16. Dispositif selon la revendication 14, caractérisé en ce que l'oligosaccharide anionique est un pentasaccharide de formule (II) 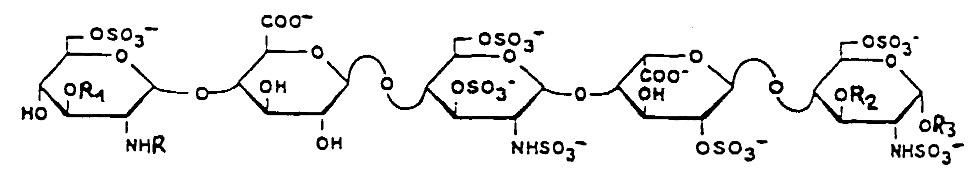 dans laquelle R est un groupement -SO₃⁻ ou acyle, notamment acétyle, R₁ et R₂, identiques ou différents, désignent H ou -SO₃⁻ et R₃ représente H ou un radical alkyle inférieur, notamment CH₃.

17. Dispositif selon l'une des revendications 1 à 16, caractérisé en ce que la quantité de principe actif présente initialement dans l'élément réservoir associé à l'électrode négative représente 1 mg à 8 mg par cm² d'électrode et par mAh de courant passant par cm² d'électrode.

18. Dispositif selon l'une des revendications 1 à 16, caractérisé en ce que l'électrode positive est une électrode réversible de même nature que l'électrode négative réversible, en ce que l'élément réservoir associé à l'électrode négative et l'élément réceptacle associé à l'électrode positive renferment chacun une quantité du principe actif de type oligosaccharidique anionique qui représente initialement 0,5 mg à 6 mg et de préférence 0,5 mg à 4 mg par cm² d'électrode et par mAh de courant passant par cm² d'électrode et en ce que le générateur de signaux électriques est agencé pour inverser la polarité des signaux électriques qu'il applique aux électrodes pour administrer le principe actif alternativement à partir de l'élément réservoir et de l'élément réceptacle.

## Claims

1. Iontophoresis device for the transcutaneous administration of an active medicament ingredient of the anionic type, which comprises a negative electrode assembly constituted by a negative electrode, known as the active electrode, in contact with a reservoir element which contains an electrolyte containing the active ingredient in an at least partially ionised form, the reservoir element being designed to ensure, when it is placed in contact with a region of a subject's skin, an ion-conductive continuum between the negative electrode and said region, a positive electrode assembly constituted either (i) by a positive electrode alone or, preferably, (ii) by a positive electrode in contact with a receptacle element containing at least one electrolyte, the receptacle element being designed to ensure, when it is placed in contact with a portion of the subject's skin, an ion-conductive continuum between the positive electrode and said portion, and a generator of electrical signals which is connectable to the two electrodes, the negative electrode in contact with the reservoir element being formed, at least in part, by an ionisable metal compound, the metal ions of which are capable of being reduced electrochemically to the corresponding metal and of forming with that metal an electrochemically reversible system, in order to constitute a reversible negative electrode, at least during the operation of the device, and the generator being designed to apply, between the electrodes, electrical signals of average voltage such that the density of the average current generated between the electrodes is between 0.03 and 0.5 mA/cm², which device is
characterised in that the active ingredient present in the reservoir element associated with the negative electrode is selected from anionic oligosaccharides represented by the alkali or alkaline earth salts of oligosaccharides which are constituted by from two to twelve saccharide units, some or all of which units have had their OH groups replaced , at least in part, by functional groups selected from -OSO₃⁻, -COO⁻, -NHSO₃⁻, -NH-acyl, -OPO₃⁻⁻ and -OT, T representing a hydrocarbon radical, and which have an ionic character suitable for iontophoretic administration, in that the current density has values of from 0.05 mA/cm² to 0.25 mA/cm² and in that the amount of active ingredient initially present in the reservoir element associated with the negative electrode represents from 0.5 to 12 mg per cm² of electrode and per mAh of current passing per cm² of electrode.

2. Device according to claim 1, characterised in that the anionic oligosaccharides are obtained by total chemical synthesis.

3. Device according to claim 1 or 2, characterised in that the metal compound capable of constituting, at least in part, the negative electrode is selected from the compounds AgCl and CuCl.

4. Device according to any one of claims 1 to 3, characterised in that the material of the negative electrode is deposited on a support, which support comprises an insulating material and especially a plastics insulating material, such as polypropylene, polyethylene, PVC, polyester, or a metal or non-metal electron-conductive material resistant to corrosion by the electrolyte containing the active ingredient in the absence of current, such as, for example, silver, titanium, platinum, stainless steel, carbon, graphite, conductive polymer.

5. Device according to claim 4, characterised in that the negative electrode is based on the pair Ag/AgCl.

6. Device according to any one of claims 1 to 5, characterised in that the positive electrode is produced from a metal or metal alloy, such as titanium, platinum, stainless steel or from a non-metal electron-conductive material, such as carbon or graphite, or consists, at least in part, of a metal capable of being consumed by electrochemical oxidation, for example a metal such as Al, Cu, Mg Zn and Ag, the metal consumable by electrochemical oxidation being in particular selected from those capable of forming an electrochemically reversible system with the metal ions resulting from electrochemical oxidation, in order to constitute a reversible positive electrode.

7. Device according to claim 6, characterised in that the positive electrode is based on the pair Ag/AgCl.

8. Device according to any one of claims 1 to 7, permitting the administration of a given total amount of the active ingredient of anionic oligosaccharide type to the subject, characterised in that the one or the other of the negative and positive electrodes is designed to constitute an electrode, known as the limiting electrode, formed by a limited amount of an electrochemically consumable material associated either with an electron-conductive support or with an insulating support, the electrochemically consumable material being either the electrochemically reducible metal compound when the limiting electrode is the negative electrode or a metal consumable by electrochemical oxidation, especially a metal such as Al, Mg, Zn and Ag, when the limiting electrode is the positive electrode, and the electron-conductive support being produced from a material which is resistant to corrosion by the electrolyte associated with the limiting electrode in the absence of current and which has, when the limiting electrode is the negative electrode, a hydrogen overpotential in the presence of the electrolyte at least equal to that of aluminium or which is not consumable by electrochemical oxidation when the limiting electrode is the positive electrode, while the limited amount of electrochemically consumable material is selected in such a manner that the amount of electricity necessary to consume it electrochemically corresponds to the amount of electricity necessary to administer the given total amount of active ingredient to the subject, so that the circulation of the current between the electrodes is in practice interrupted when the consumable material of the limiting electrode has been consumed, and in that the active ingredient of anionic oligosaccharide type is present, at the beginning of the operation, in the reservoir element in contact with the negative electrode in an amount higher than the given total amount to be administered to the subject.

9. Device according to any one of claims 1 to 8, characterised in that the generator of electric signals applies between the negative electrode and the positive electrode an intensiometric signal, that is to say, a signal of set average intensity, or a potentiometric signal, that is to say, a signal of set average voltage, the electric signal being continuous or pulsed and permanent or intermittent, with or without temporary inversion of polarity, and having a frequency of from 0 to 500 kHz and more particularly from 0 to 100 kHz.

10. Device according to claim 9, characterised in that the electrical signal is a pulsed signal having a cyclic ratio, that is to say, a ratio of the duration of the elementary pulse, the repetition of which forms the pulsed signal, to the time interval separating two consecutive appearances of that pulse, of from 0.05 to 0.95 and more particularly from 0.1 to 0.8.

11. Device according to claim 9 or 10, characterised in that the signal applied between the negative electrode and the positive electrode has an average voltage selected between 0.1 and 50 volts and more especially between 0.5 and 20 volts, so that the density of the average current generated between the electrodes has a value of between 0.05 and 0.25 mA/cm² and more particularly between 0.05 and 0.2 mA/cm².

12. Device according to any one of claims 1 to 11, characterised in that the aqueous medium containing the active ingredient of anionic oligosaccharide type and/or the aqueous medium constituting the other electrolyte contain agents capable of promoting the transcutaneous passage of the active ingredient, such as, for example, vasodilator agents and/or amphiphilic agents, such as, especially, compounds of the alcohol type or of the ester type.

13. Device according to any one of claims 1 to 12, characterised in that the active ingredient of anionic oligosaccharide type present in the reservoir element associated with the negative electrode is selected from the alkali or alkaline earth salts, especially salts of sodium, potassium or calcium, of oligosaccharides which are constituted by from three to eight saccharides units, some or all of which units have had their OH groups replaced, at least in part, by functional groups such as, for example, -OSO₃⁻, -COO⁻, -NHSO₃⁻, -NH-acyl, -OPO₃⁻⁻ and -OT where T represents a hydrocarbon radical and especially an aliphatic or aromatic hydrocarbon radical, -OT being in particular an alkoxy group, and which have an ionic character suitable for iontophoretic administration, the oligosaccharides being more especially anionic oligosaccharides obtained by total chemical synthesis.

14. Device according to claim 13, characterised in that the anionic oligosaccharides are tri-, tetra-, penta- or hexasaccharides and very particularly pentasaccharides.

15. Device according to any one of claims 1 to 14, characterised in that the anionic oligosaccharides are constituted by uronic acid units alternating with glucosamine units or by uronic acid units alternating with glucose units or by uronic acid units alternating with galactoseamine units.

16. Device according to claim 14, characterised in that the anionic oligosaccharide is a pentasaccharide of formula (II) 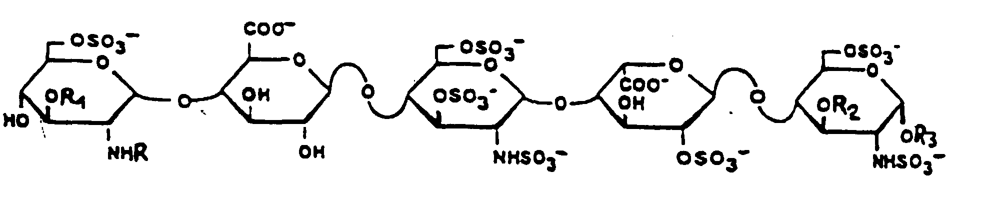 wherein R is a -SO₃⁻ or acyl group, especially acetyl, R₁ and R₂, which may be the same or different, denote H or -SO₃⁻ and R₃ represents H or a lower alkyl radical, especially CH₃.

17. Device according to any one of claims 1 to 16, characterised in that the amount of active ingredient initially present in the reservoir element associated with the negative electrode represents from 1 mg to 8 mg per cm² of electrode and per mAh of current passing per cm² of electrode.

18. Device according to any one of claims 1 to 16, characterised in that the positive electrode is a reversible electrode of the same nature as the reversible negative electrode, in that the reservoir element associated with the negative electrode and the receptacle element associated with the positive electrode each contain an amount of active ingredient of anionic oligosaccharide type which represents initially from 0.5 mg to 6 mg and preferably from 0.5 mg to 4 mg per cm² of electrode and per mAh of current passing per cm² of electrode and in that the generator of electrical signals is designed to invert the polarity of the electrical signals which it applies to the electrodes in order to administer the active ingredient alternately from the reservoir element and from the receptacle element.

## Patentansprüche

1. Ionophoresevorrichtung zur transkutanen Verabreichung eines Arzneimittelwirkstoffs vom anionischen Typ, die eine negative Elektrodeneinheit, die aus einer negativen Elektrode, der sogenannten aktiven Elektrode, im Kontakt mit einem ersten Behälter besteht, der einen Elektrolyten enthält, der seinerseits den Wirkstoff in wenigstens teilweise ionisierter Form enthält, wobei dieser Behälter so beschaffen ist, dass beim Kontakt mit einer Hautzone des Patienten zwischen der negativen Elektrode und der Zone ein ionisches Leiterkontinuum gebildet wird, eine positive Elektrodeneinheit, die entweder (i) aus einer positiven Elektrode allein oder vorzugsweise (ii) aus einer positiven Elektrode im Kontakt mit einem zweiten Behälter besteht, der wenigstens einen Elektrolyten enthält und so beschaffen ist, dass er beim Kontakt mit einem Teil der Haut des Patienten ein ionisches Leiterkontinuum zwischen der positiven Elektrode und dem Hautanteil gewährleistet, und eine elektrische Signalerzeugungseinrichtung umfasst, die mit den beiden Elektroden verbunden werden kann, wobei die negative Elektrode im Kontakt mit dem ersten Behälter zumindest teilweise aus einer ionisierbaren Metallverbindung gebildet ist, deren Metallionen elektrochemisch zum entsprechenden Metall reduziert werden können und mit dem Metall ein elektrochemisch reversibles System zu bilden vermögen, um zumindest während des Betriebs der Vorrichtung eine reversible negative Elektrode zu bilden, und die Signalerzeugungseinrichtung so beschaffen ist, dass sie zwischen den Elektroden elektrische Signale von durchschnittlicher Spannung setzt, so dass die Dichte des zwischen den Elektroden erzeugten durchschnittlichen Stroms einen Wert von 0,03 bis 0,5 mA/cm² aufweist, **dadurch gekennzeichnet,** dass der in dem mit der negativen Elektrode verbundenen ersten Behälter enthaltene Wirkstoff ausgewählt ist unter anionischen Oligosacchariden wie Alkali- oder Erdalkalimetallsalzen von Oligosacchariden, die aus zwei bis zwölf Saccharidfragmenten bestehen, wobei bei einigen dieser Fragmente oder bei allen die OH-Gruppen zumindest teilweise durch funktionelle Gruppen, ausgewählt unter -OSO₃⁻, -COO⁻, -NHSO₃⁻, -NH-Acyl, -OPO₃⁻⁻ und -OT, worin T einen Kohlenwasserstoffrest bedeutet, ersetzt sind, und einen ionischen Charakter aufweisen, der für eine ionophoretische Verabreichung geeignet ist, die Stromdichte Werte von 0,05 bis 0,25 mA/cm² aufweist und die anfänglich in dem mit der negativen Elektrode verbundenen ersten Behälter enthaltene Wirkstoffmenge 0,5 bis 12 mg pro cm² Elektrode und pro mAh des pro cm² Elektrode hindurchtretenden Stroms beträgt.

2. Vorrichtung nach Anspruch 1, **dadurch gekennzeichnet,** dass die anionischen Oligosaccharide durch chemische Gesamtsynthese erhalten wurden.

3. Vorrichtung nach Anspruch 1 oder 2, **dadurch gekennzeichnet,** dass die Metallverbindung, die wenigstens teilweise die negative Elektrode zu bilden vermag, unter den Verbindungen AgCl und CuCl ausgewählt wurde.

4. Vorrichtung nach einem der Ansprüche 1 bis 3, **dadurch gekennzeichnet,** dass der Stoff der negativen Elektrode auf einen Träger abgeschieden wurde, der aus einem isolierenden Material und insbesondere einem isolierenden Kunststoff wie Polypropylen, Polyethylen, PVC, Polyester oder aus einem metallischen oder nichtmetallischen elektronisch leitenden Stoff, der der Korrosion durch den Elektrolyten, welcher den Wirkstoff enthält, wenn kein Strom fließt, zu widerstehen vermag, wie z.B. Silber, Titan, Platin, nichtrostender Stahl, Ruß, Graphit oder leitfähiges Polymer, besteht.

5. Vorrichtung nach Anspruch 4, **dadurch gekennzeichnet,** dass die negative Elektrode auf der Basis des Paares Ag/AgCl beruht.

6. Vorrichtung nach einem der Ansprüche 1 bis 5, **dadurch gekennzeichnet,** dass die positive Elektrode aus einem Metall oder einer Metallegierung wie Titan, Platin oder nichtrostendem Stahl oder einem nichtmetallischen elektronisch leitenden Stoff wie Ruß oder Graphit oder zumindest teilweise aus einem Metall besteht, das durch elektrochemische Oxidation zersetzt werden kann, wie z.B. Al, Cu, Mg, Zn und Ag, wobei das durch elektrochemische Oxidation zersetzbare Metall insbesondere unter Metallen ausgewählt wird, die mit den sich aus der elektrochemischen Oxidation ergebenden Metallionen ein elektrochemisch reversibles System zu bilden vermögen, um auf diese Weise eine reversible positive Elektrode zu bilden.

7. Vorrichtung nach Anspruch 6, **dadurch gekennzeichnet,** dass die positive Elektrode auf der Basis des Paares Ag/AgCl beruht.

8. Vorrichtung nach einem der Ansprüche 1 bis 7, welche die Verabreichung einer gegebenen Gesamtmenge an Wirkstoff vom Typ eines anionischen Oligosaccharids an einen Patienten ermöglicht, **dadurch gekennzeichnet,** dass die negative oder positive Elektrode so beschaffen ist, dass sie eine Elektrode, die sogenannte Begrenzungselektrode, bildet, die aus einer begrenzten Menge eines elektrochemisch zersetzbaren Stoffes besteht, der mit einem elektronisch leitenden Träger oder einem isolierenden Träger verbunden ist und eine elektrochemisch reduzierbare Metallverbindung darstellt, wenn die Begrenzungselektrode die negative Elektrode ist, oder ein durch elektrochemische Oxidation zersetzbares Metall, insbesondere Al, Mg, Zn und Ag, darstellt, wenn die Begrenzungselektrode die positive Elektrode ist, und der elektronisch leitende Träger aus einem Material hergestellt ist, das der Korrosion durch den mit der Begrenzungselektrode verbundenen Elektrolyten, wenn kein Strom fließt, zu widerstehen vermag und, wenn die Begrenzungseleketrode die negative Elektrode ist, in Anwesenheit des Elektrolyten eine Wasserstoffüberspannung aufweist, die zumindest der des Aluminiums gleichkommt, oder, wenn die Begrenzungselektrode die positive Elektrode ist, durch elektrochemische Oxidation nicht zersetzbar ist, während die begrenzte Menge an elektrochemisch zersetzbarem Stoff so ausgewählt wird, dass die für seine elektrochemische Zersetzung erforderliche Strommenge derjenigen entspricht, die erforderlich ist, um dem Patienten die gegebene Gesamtmenge an Wirkstoff zu verabreichen, so dass der Stromkreis zwischen den Elektroden praktisch unterbrochen wird, sobald der zersetzbare Stoff der Begrenzungselektrode zersetzt ist, und dass der Wirkstoff vom Typ eines anionischen Oligosaccharids zu Beginn des Vorgangs im ersten Behälter im Kontakt mit der negativen Elektrode in einer höheren Menge vorliegt als der dem Patienten zu verabreichenden vorgegebenen Gesamtmenge entspricht.

9. Vorrichtung nach einem der Ansprüche 1 bis 8, **dadurch gekennzeichnet**, dass die elektrische Signalerzeugungseinrichtung zwischen der negativen und der positiven Elektrode ein intensiometrisches Signal, d.h. ein Signal mit einer erzwungenen durchschnittlichen Stärke, oder ein potentiometrisches Signal, d.h. ein Signal mit einer erzwungenen durchschnittlichen Spannung, setzt, wobei das elektrische Signal kontinuierlich oder pulsierend bzw. permanent oder unterbrochen sein kann, mit oder ohne zeitweise Umpolung und mit einer Frequenz zwischen 0 und 500 kHz und insbesondere zwischen 0 und 100 kHz.

10. Vorrichtung nach Anspruch 9, **dadurch gekennzeichnet,** dass das elektrische Signal ein gepulstes Signal ist, das ein zyklisches Verhältnis, d.h. ein Verhältnis zwischen der Dauer des Einzelimpulses, dessen Wiederholung das gepulste Signal bildet, und dem Zeitintervall zwischen zwei aufeinanderfolgenden Impulsen von 0,05 bis 0,95 und insbesondere von 0,1 bis 0,8 aufweist.

11. Vorrichtung nach Anspruch 9 oder 10, **dadurch gekennzeichnet,** dass das zwischen der negativen Elektrode und der positiven Elektrode gesetzte Signal eine durchschnittliche Spannung zwischen 0,1 und 50 Volt und insbesondere zwischen 0,5 und 20 Volt hat, so dass die Dichte des zwischen den Elektroden erzeugten durchschnittlichen Stroms einen Wert von 0,05 bis 0,25 mA/cm² und insbesondere von 0,05 bis 0,2 mA/cm² aufweist.

12. Vorrichtung nach einem der Ansprüche 1 bis 11, **dadurch gekennzeichnet,** dass das den Wirkstoff vom Typ eines anionischen Oligosaccharids enthaltende wässrige Medium und/oder das den anderen Elektrolyten bildende wässrige Medium Stoffe enthalten, welche die transkutane Passage des Wirkstoffs zu begünstigen vermögen, wie z.B. gefäßerweiternde und/oder amphiphile Mittel, wie z.B. Verbindungen vom Alkohol- oder Estertyp.

13. Vorrichtung nach einem der Ansprüche 1 bis 12, **dadurch gekennzeichnet,** dass der in dem mit der negativen Elektrode verbundenen ersten Behälter vorliegende Wirkstoff vom Typ eines anionischen Oligosaccharids unter Alkali- oder Erdalkalimetallsalzen, insbesondere unter Natrium-, Kalium- oder Calciumsalzen von Oligosacchariden ausgewählt wird, die aus drei bis acht Saccharidfragmenten gebildet sind, wobei bei einigen dieser Fragmente oder bei allen die OH-Gruppen zumindest teilweise durch funktionelle Gruppen, wie z.B. -OSO₃, -COO⁻, -NHSO₃⁻, -NH-Acyl, -OPO₃⁻⁻ und -OT, worin T einen Kohlenwasserstoffrest und insbesondere einen aliphatischen oder aromatischen Kohlenwasserstoffrest darstellt und -OT insbesondere eine Alkoxygruppe darstellt, ersetzt sind, und die einen für eine ionophoretische Verabreichung geeigneten ionischen Charakter aufweisen, wobei die Oligosaccharide insbesondere durch chemische Gesamtsynthese erhaltene anionische Oligosaccharide sind.

14. Vorrichtung nach Anspruch 13, **dadurch gekennzeichnet,** dass die anionischen Oligosaccharide Tri-, Tetra-, Penta- oder Hexasaccharide und insbesondere Pentasaccharide sind.

15. Vorrichtung nach einem der Ansprüche 1 bis 14, **dadurch gekennzeichnet,** dass die anionischen Oligosaccharide aus alternierenden Uronsäure- und Glucosaminfragmenten oder aus alternierenden Uronsäure- und Glucosefragmenten oder aus alternierenden Uronsäure- und Galactosaminfragmenten aufgebaut sind.

16. Vorrichtung nach Anspruch 14, **dadurch gekennzeichnet,** dass das anionische Oligosaccharid ein Pentasaccharid der Formel (II) ist 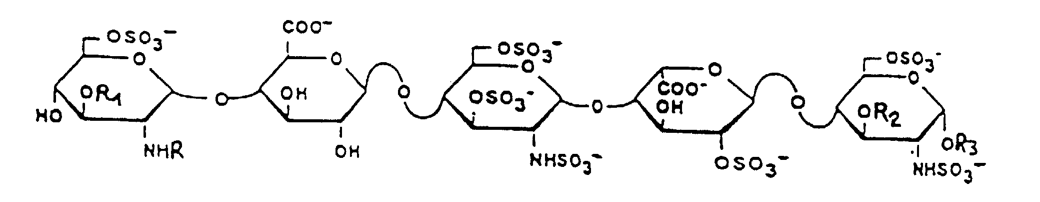 worin R eine Gruppe -SO₃⁻ oder Acyl, insbesondere Acetyl, ist, R₁ und R₂ gleich oder verschieden sind und H oder -SO₃⁻ bedeuten und R₃ H oder einen Niederalkylrest, insbesondere CH₃, bedeutet.

17. Vorrichtung nach einem der Ansprüche 1 bis 16, **dadurch gekennzeichnet,** dass die Menge an Wirkstoff, die zu Beginn in dem mit der negativen Elektrode verbundenen ersten Behälter enthalten ist, 1 bis 8 mg pro cm² Elektrode und pro mAh des pro cm² Elektrode hindurchtretenden Stroms beträgt.

18. Vorrichtung nach einem der Ansprüche 1 bis 16, **dadurch gekennzeichnet,** dass die positive Elektrode eine reversible Elektrode derselben Art wie die reversible negative Elektrode ist und dass der mit der negativen Elektrode verbundene erste Behälter und der mit der positiven Elektrode verbundene zweite Behälter jeweils eine Menge an Wirkstoff vom Typ eines anionischen Oligosaccharids enthalten, die zu Beginn 0,5 bis 6 mg und vorzugsweise 0,5 bis 4 mg pro cm² Elektrode und pro mAh des pro cm² Elektrode hindurchtretenden Stroms beträgt und die elektrische Signalerzeugungseinrichtung so beschaffen ist, dass sie die Polarität der elektrischen Signale, die sie zwischen die Elektroden zur Verabreichung des Wirkstoffs alternierend ausgehend vom ersten Behälter und zweiten Behälter umzukehren vermag.
